# EUROPEAN PATENT APPLICATION

(11) **EP 3 346 253 A1**
(43) Date of publication of application: **11.07.2018**
(21) Application number: 18000006.9
(22) Date of filing: 04.01.2018
(51) Int. Cl.: G01N 1/22

(54) **DYNAMIC FLUX CHAMBER FOR MONITORING AND SAMPLING SOIL GAS FROM AN INDOOR OR OUTDOOR BREATHABLE SURFACE**

(30) Priority: 05.01.2017 IT 201700001216; 06.04.2017 IT 201700038294
(71) Applicant: Milone, Elio, 10155 Torino (TO) (IT)
(72) Inventor: Milone, Elio, 10155 Torino (TO) (IT)
(74) Representative: Aprà, Mario

(57) **Abstract**

Dynamic flux chamber (10) for monitoring and sampling soil gas from an indoor or outdoor breathable surface (S1), comprising:
- a body (11) including an internal cavity (12) for collecting soil gas and having a mouth (13) overlaid on said breathable surface (S1);
- a pipe (15) for feeding pressurized inert gas at least into said internal cavity (12), coming from an external source of pressurized inert gas;
- a plurality of apertures (16.1, 16.2, 16.3, 16.4) provided in said body (11), both for the inflow of inert gas through said pipe (15), and for the outflow of excess inert gas and/or for parameter control during monitoring and/or for sampling the test gas collected in said cavity (12), and a seal (14) juxtaposed against said mouth (13) and resting against said breathable surface (S1).

## Description

The present invention relates to a dynamic flux chamber for monitoring and sampling soil gas from an indoor or outdoor breathable surface. The expression "soil gas" used here refers to gases / vapours coming from the soil or subsoil, and for which one wishes to assess the flow, for example, in terms of mass per surface unit over a unit of time.

The present invention also relates to a procedure for monitoring and sampling outdoor or indoor soil gas, using said flux chamber.

Several types of means for monitoring outdoor or open-air soil gases are known, called, for example, dynamic or static Soil Gas and Flux Chambers.

Sampling using so-called Soil Gas technology is done by means of a sampling pit, achieved by digging a deep hole of as much as several meters. This hole can obviously be made at outdoor sampling points, but it cannot be used for a preliminary assessment as part of an initial inspection due to the complexity involved. It can therefore only be used once a real requirement for long-term control has been defined. The Soil Gas sampling point is also influenced by climatic conditions during the test period. For example, when sampling at a Soil Gas point is conducted in the winter, after a period of rain, it is probable that ice has formed inside the sampling pit or there may be residues of water, thereby making it temporarily impossible to conduct sampling.

Sampling using so-called static Flux Chamber technology for outdoor soil gas is done by positioning the chamber on the ground, without external intervention of any kind, for long periods, possibly even several days, so that the soil gas accumulates inside the chamber by means of natural flow. Using this system, it is not possible to ascertain whether the inside of the chamber is influenced by the outside air, when the sampling time becomes extremely long.

Sampling using so-called dynamic Flux Chamber technology for outdoor soil gas is done by means of the continuous insufflation of inert gas into the chamber, using known procedures and technologies, which do not guarantee the homogeneity of gas throughout the flux chamber.

Moreover, it is fundamental to know whether there are polluted soil gases on a site, whether industrial or not, especially in the case of reclamation and in order to estimate related hygiene and health risks.

An object of the present invention is to provide a dynamic flux chamber for monitoring and sampling soil gas from an indoor or outdoor breathable surface, which makes it possible to monitor the soil gases coming from the outdoor or indoor soil/subsoil, without damaging the sampling point.

A further object of the present invention is to provide a flux chamber and relative procedure for monitoring and sampling soil gas from an indoor or outdoor breathable surface, that is simple, safe and reliable to conduct and can guarantee the insufflation of inert gas in a homogeneous manner throughout the chamber, thereby aiding in the extraction of the soil gas in a homogeneous manner over the entire surface on which the flux chamber is placed, without acting permanently and/or invasively in the testing area, and guaranteeing a realistic measurement of the soil gas.

In light of the afore-mentioned objects, the present invention provides a dynamic flux chamber for monitoring and sampling soil gas from an indoor or outdoor breathable surface, comprising:
- a body including an internal cavity for collecting soil gas and having a mouth overlaid on said breathable surface,
- a seal juxtaposed against said mouth and resting against said breathable surface;
- a pipe for feeding pressurized inert gas at least into said internal cavity, coming from an external source of pressurized inert gas;
- a plurality of apertures provided in said body, both for the inflow of inert gas through said pipe, and for the outflow of excess inert gas and/or for parameter control during monitoring and/or for sampling the test gas collected in said cavity.

The dependent claims relate to particular embodiments of the present invention.

In particular, said dynamic flux chamber, according to the invention, comprises an at least partial gap provided between an outer wall and an inner wall of said body. Said pipe comprises a first duct for feeding pressurized inert gas in a gas-tight manner from said external source of pressurized inert gas into said cavity of said body and a second duct feeding pressurized inert gas into said cavity and/or into said gap. Said inner wall of said body comprises at least one through-hole which creates a mutual gas communication between said gap and said cavity. Furthermore, said second duct connects in a gas-tight manner said first duct with said gap and has at least one through-hole for the passage of pressurized inert gas into said cavity of said body.

It will be noted that thanks to the combined presence of said at least one through-hole, which creates a mutual gas communication between said gap and said cavity, and of said at least one through-hole provided in said second duct, the insufflation of inert gas into said flux chamber takes place in a homogeneous manner, throughout the internal cavity, and aids the accumulation of soil gas in a homogeneous manner, throughout said internal cavity.

Additionally, said body of said flux chamber comprises:
- a dome including:
   - a top cover, which has said plurality of apertures;
   - said outer wall;
   - said inner wall, wherein are provided a plurality of small-diameter through-holes and two through-holes of a larger diameter;
   - a continuous peripheral projection of said cover, engaged with the upper zone of at least said outer wall and/or said inner wall;
   - a tubular organ comprising said second duct, having a plurality of small-diameter radial through-holes and a gas-tight radial hole connecting said first duct, said tubular organ being arranged with its axial ends in said through-holes of a larger diameter provided in said gas-tight inner wall, providing a tunnel for inert gas to flow into said gap.

Furthermore, said flux chamber is lined on the outside with a thermally insulating covering that also blocks UV radiation. Said body and said frame provide a kit of components, facilitating the commissioning and decommissioning of said flux chamber, even by an inexpert person.

The present invention also provides a procedure for monitoring and sampling soil gas from an outdoor or indoor breathable surface, actuated by means of the dynamic flux chamber described above, wherein:
- said seal is placed against said breathable surface of an outdoor or indoor environment;
- said body is placed in contact with said seal juxtaposing the edge of said mouth against the seal;
- an inert gas is blown continuously through said pipe, passing through a hole of said body of the flux chamber, into said cavity of the body, coming from an external source of pressurized inert gas;
- soil gas present in the test area is extracted, and is collected inside said cavity of the body of the flux chamber and then sampled in specific substrates.

Furthermore, according to the afore-mentioned procedure:
- the inert gas is channelled through said second duct, provided with small holes, and flows out of said small holes, penetrating into said cavity of the body of the flux chamber,
- residual inert gas is further channelled towards and as far as said gap between said outer wall and said inner wall of said body, provided with small holes, and flows out through said small holes, penetrating into the internal cavity of the chamber.

Other characteristics and advantages of the invention will become more apparent from the following detailed description of two examples of embodiments of the invention, with reference to the accompanying drawings, which show important details of the invention, and from the claims. The characteristics illustrated here must not necessarily be taken as drawn to scale, but rather are represented in such a way that the peculiarities according to the invention are clearly highlighted. The different characteristics can be produced singly or in any combination thereof, as variants of the invention.

In the accompanying drawings:
- fig. 1 shows an elevation view of a first example of an embodiment of the dynamic flux chamber for monitoring and sampling soil gas from an indoor or outdoor breathable surface according to the invention;
- fig. 2 shows a perspective, exploded view of the flux chamber shown in fig. 1;
- fig. 3 shows an overhead plan view of the flux chamber according to the arrow III in fig. 1;
- fig. 4 shows a vertical cross-section view along the line IV-IV in Fig. 5;
- fig. 5 shows a cross-section view along the line V-V in fig. 3, provided with a duct for feeding inert gas;
- fig. 6 shows a cross-section view along the line VI-VI in fig. 4;
- figures 6a to 6d show views of the detail A in fig. 6, being plan and cross-section views respectively, illustrating the modular means of connecting parts of said camera, in a closed and open position;
- fig. 7 shows a perspective, partial cut-away view, in a different scale, of a pipe of inert gas for the flux chamber according to the previous figures;
- fig. 8 shows a cross-section view along the line VIII-VIII in fig. 7;
- fig. 9 shows a cross-section view along the line IX-IX in fig. 8;
- fig. 10 shows a view of the flux chamber according to the previous figures in operation, with ducts inserted for the introduction of inert gas, for the outflow of excess inert gas and/or for parameter control during monitoring and/or for sampling the test gas collected in the cavity of said chamber, said chamber being protected by means of a cover made of polystyrene or similar;
- fig. 11 shows a front three-quarter overhead perspective view of a second example of an embodiment of the dynamic flux chamber for monitoring and sampling indoor soil gas according to the invention, provided with a duct for feeding inert gas;
- fig. 12 shows a perspective, exploded view of the flux chamber according to fig. 11;
- fig. 13 shows a rear overhead perspective view of the flux chamber according to fig. 11;
- fig. 14 shows an overhead plan view according to the arrow XIV in fig. 13;
- fig. 15 shows a cross-section view along the line XV-XV in fig. 14, provided with a duct for feeding inert gas;
- fig. 16 shows a cross-section view along the line XVI-XVI in fig. 15;
- fig. 17 shows a perspective, partial cut-away view, in a different scale, of a pipe of inert gas for the flux chamber according to the previous figures;
- fig. 18 shows a cross-section view along the line XVIII-XVIII in fig. 17;
- fig. 19 shows a cross-section view along the line XIX-XIX in fig. 18;
- fig. 20 shows a view of the flux chamber according to the previous figures in operation, with ducts inserted for the introduction of inert gas, the outflow of excess inert gas and/or for parameter control during monitoring and/or for sampling the test gas collected in the cavity of said chamber, said chamber being protected by means of a cover made of polystyrene or similar.

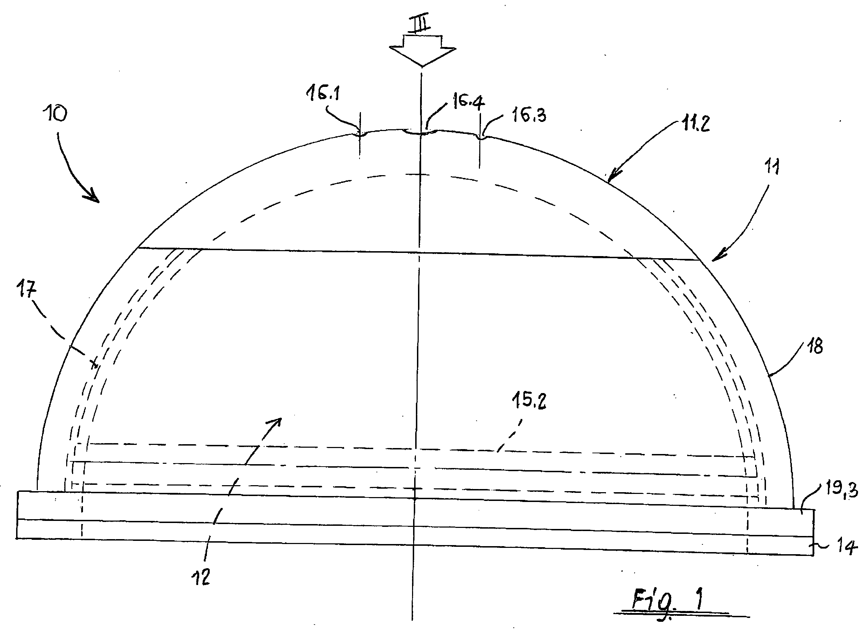

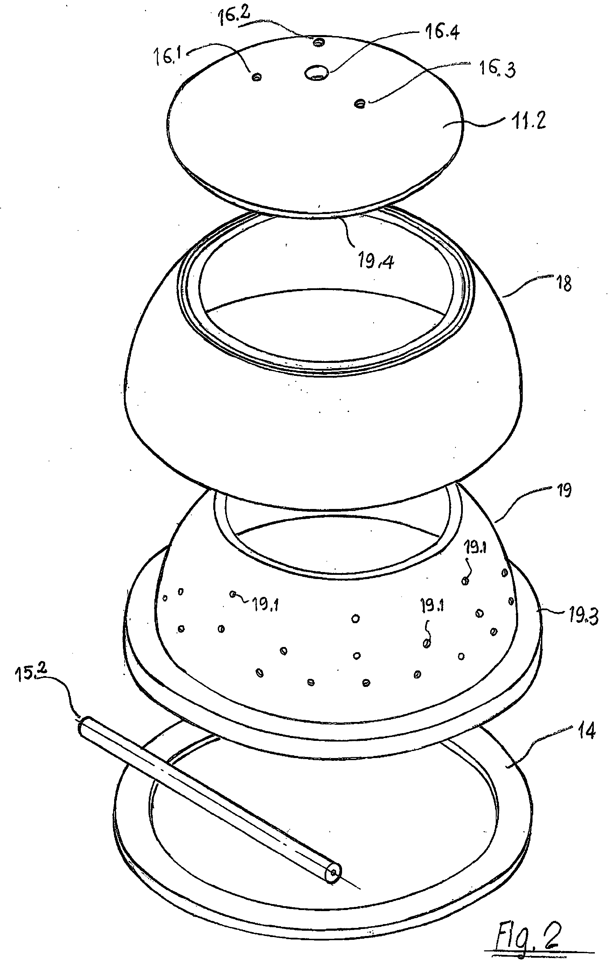

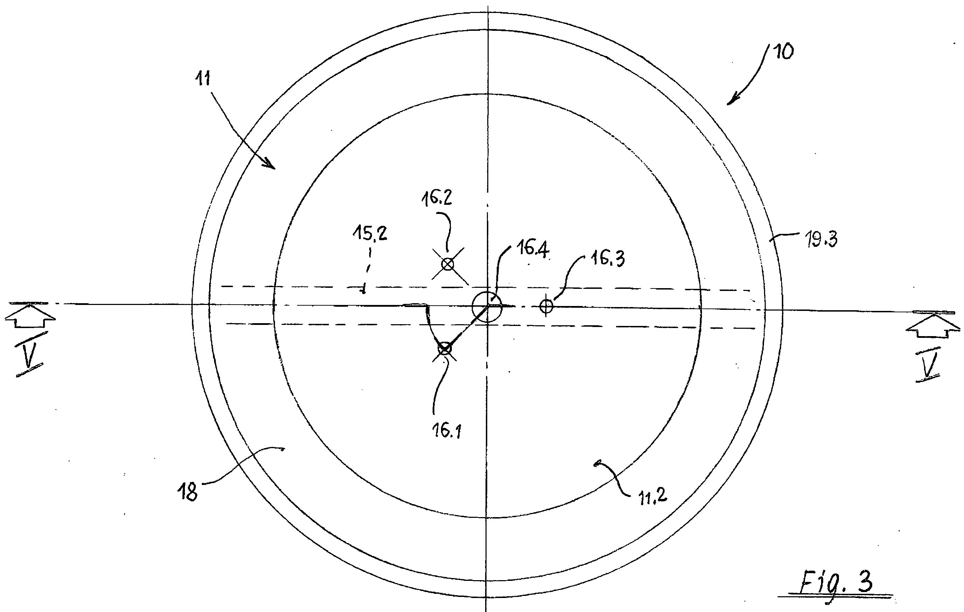

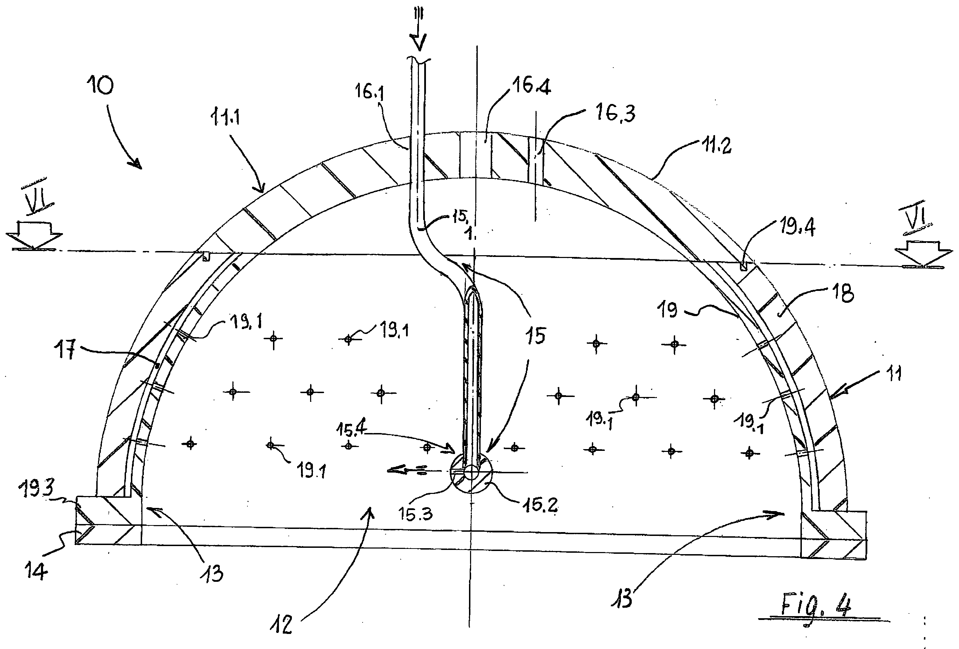

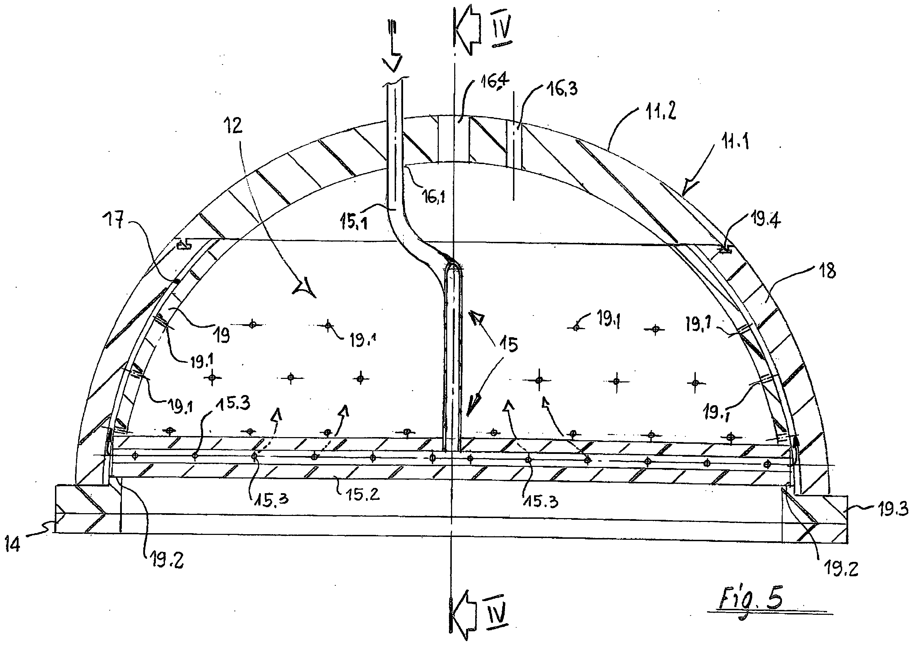

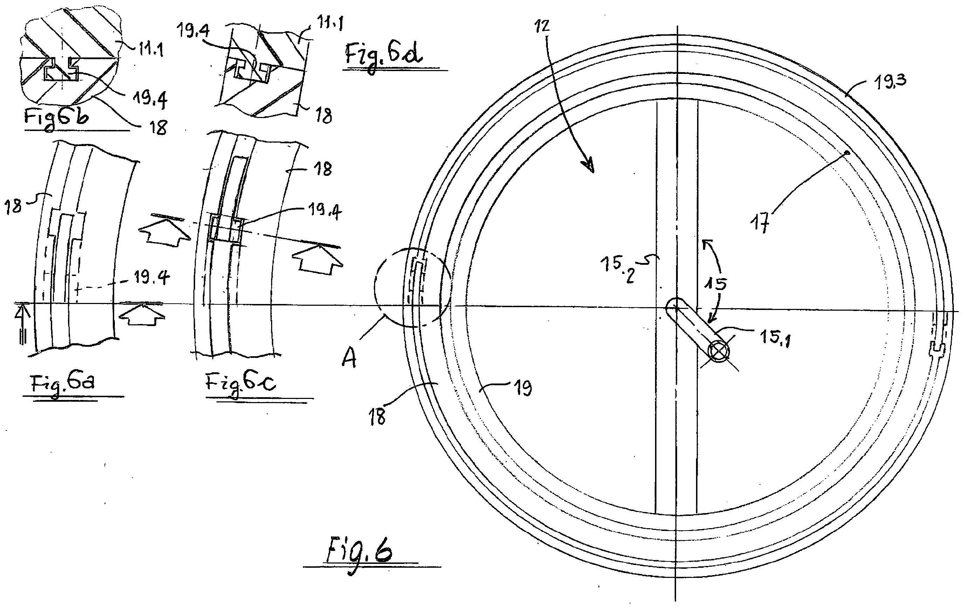

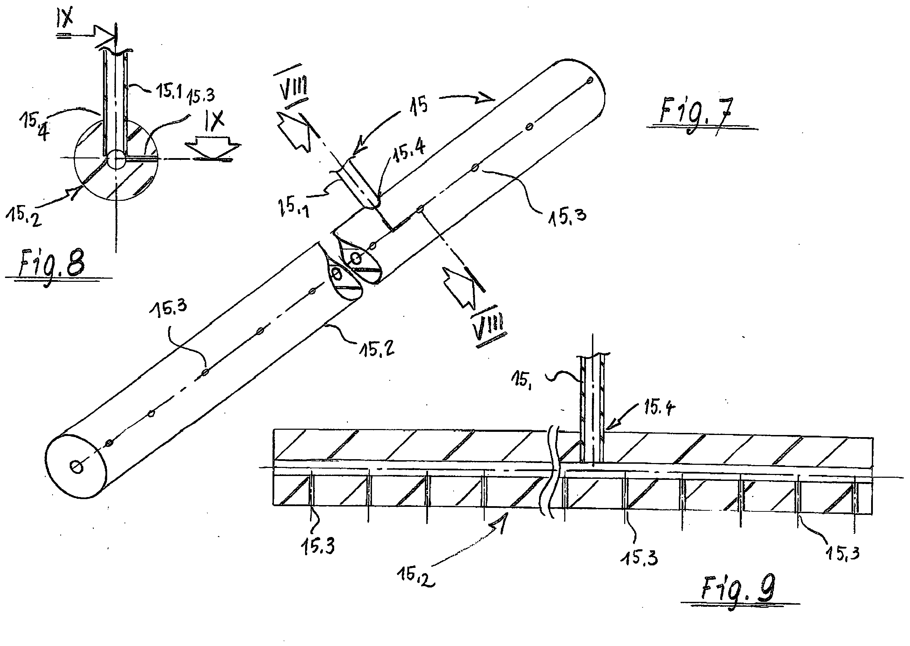

### First example of embodiment (figures 1 to 10)

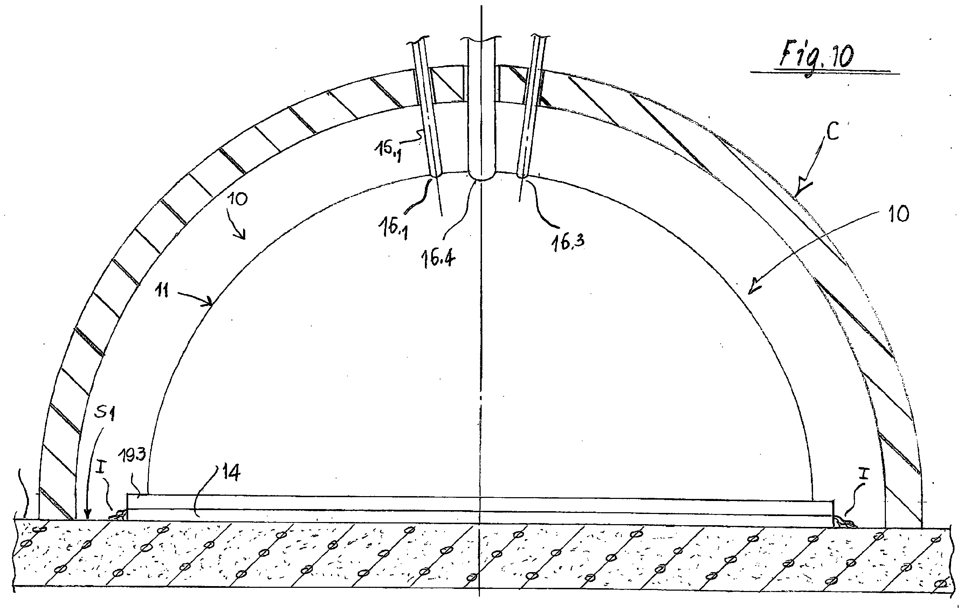

With reference to the accompanying drawings, the number 10 is used to refer to the entire dynamic flux chamber for monitoring and sampling soil gas from an indoor or outdoor breathable surface S1 (fig. 10), according to an example of an embodiment of the present invention.

In particular, said flux chamber 10 comprises:
- a body 11 including an internal cavity 12 for collecting soil gas and having a mouth 13 overlaid on said breathable surface S1,
- a seal 14 juxtaposed against said mouth 13 and resting against said breathable surface S1;
- a pipe 15 for feeding pressurized inert gas at least into said internal cavity 12, coming from an external source of pressurized inert gas (not illustrated);
- a plurality of apertures 16.1, 16.2, 16.3, 16.4 provided in said body 11, both for the inflow of inert gas through said pipe 15, and for the outflow of excess inert gas and/or for parameter control during monitoring and/or for sampling the test gas collected in said cavity 12.

Furthermore, said flux chamber comprises an at least partial gap 17 provided between an outer wall 18 and an inner wall 19 of said body 11. Said pipe 15 comprises a first duct 15.1 for feeding pressurized inert gas in a gas-tight manner from said external source of pressurized inert gas into said cavity 12 of said body 11 and a second duct 15.2 feeding pressurized inert gas into said cavity 12 and/or into said gap 17.

Said inner wall 19 of said body 11 comprises at least one through-hole 19.1, which creates a mutual gas communication between said gap 17 and said cavity 12. Conveniently, said second duct 15.2 connects in a gas-tight manner said first duct 15.1 with said gap 17 and has at least one through-hole 15.3 for the passage of pressurized inert gas into said cavity 12 of said body 11.

Additionally, said body 11 of said flux chamber 10 comprises:
- a dome 11.1 including:
   - a top cover 11.2, which has said plurality of apertures 16.1, 16.2, 16.3, 16.4;
   - said outer wall 18;
   - said inner wall 19, wherein are provided a plurality of small-diameter through-holes 19.1 and two through-holes 19.2 of a larger diameter;
   - a continuous peripheral projection 19.4 of said cover 11.2, engaged with the upper zone of at least said outer wall 18;
   - a tubular organ 15.2 comprising said second duct, having a plurality of small-diameter radial through-holes 15.3 and a gas-tight radial hole 15.4 connecting said first duct 15.1, said tubular organ 15.2 being arranged with its axial ends in said through-holes 19.2 of a larger diameter provided in said gas-tight inner wall 19, providing a tunnel for inert gas to flow into said gap 17.

Furthermore, said seal 14 has a circular frame shape, against which rests at least said inner wall 19, by means of a continuous circular base 19.3.

Said body is lined on the outside with a thermally insulating covering (C, fig. 10) that also blocks UV radiation.

Advantageously, said chamber 10 comprises inert material (I) not containing any chemical substance present in the test gas, said inert material (I) being arranged along the outer edge of said seal 14, so that said inert material (I) is not completely insulating and sealing.

Said body 11 and said frame 14 provide a kit of components, facilitating the commissioning and decommissioning of said flux chamber 10, even by an inexpert person.

Procedure for monitoring and sampling soil gas from an indoor or outdoor breathable surface, actuated by means of said dynamic flux chamber 10.

Said procedure comprises the following steps:
- said seal 14 is placed against said breathable surface S1 of an outdoor or indoor environment;
- said body 11 is placed in contact with said seal 14 juxtaposing said edge 19.3 of said mouth 13 against the seal;
- an inert gas is blown continuously through said pipe 15, 15.1, 15.2, passing through a hole 16.1 of said body 11, 11.1, 11.2 of the flux chamber 10, into said cavity 12 of the body, coming from an external source of pressurized inert gas;
- soil gas present in the test area is extracted, and is collected inside said cavity 12 of the body 11, 11.1, 11.2 of the flux chamber and then sampled in specific substrates. Furthermore, according to said procedure:
- the inert gas is channelled through said second duct 15.2, provided with small holes 15.3 and partly flows out of said small holes 15.3, penetrating into said cavity 12 of the body 11, 11.1, 11.2 of the flux chamber 10,
- residual inert gas is further channelled towards and as far as said gap 17 between said outer wall 18 and said inner wall 19 of said body, provided with small holes 19.1, and flows out through said small holes 19.1, penetrating into the internal cavity 12 of the chamber.

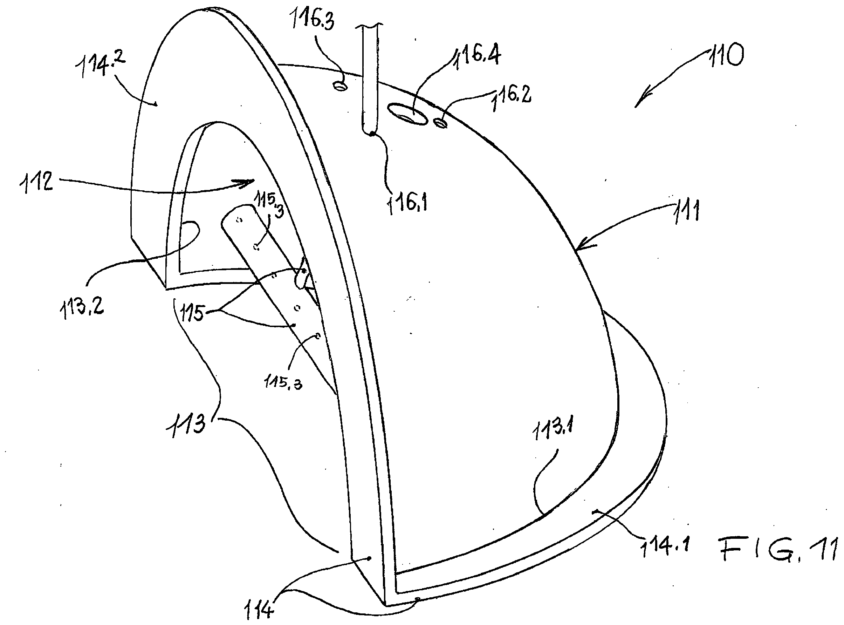

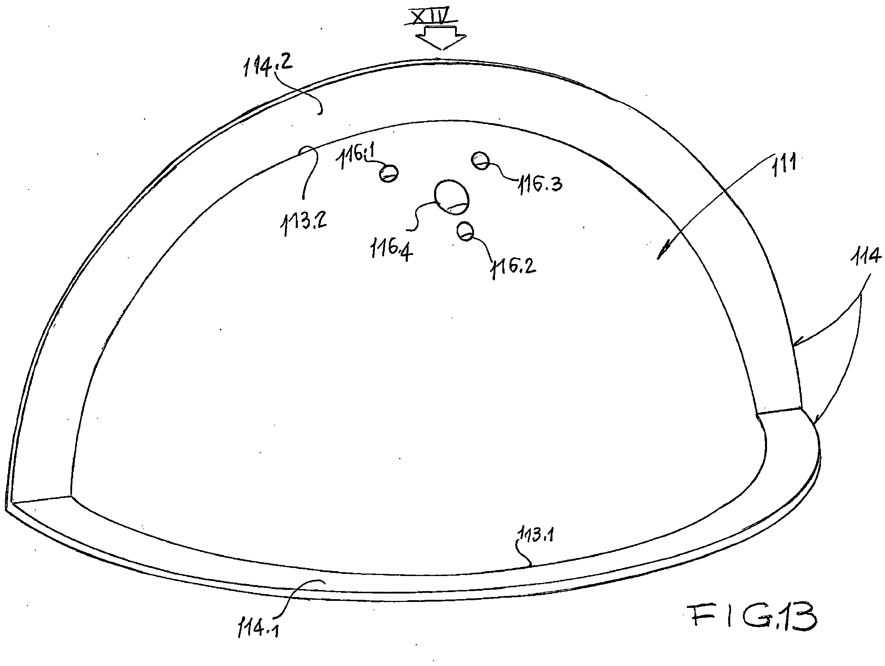

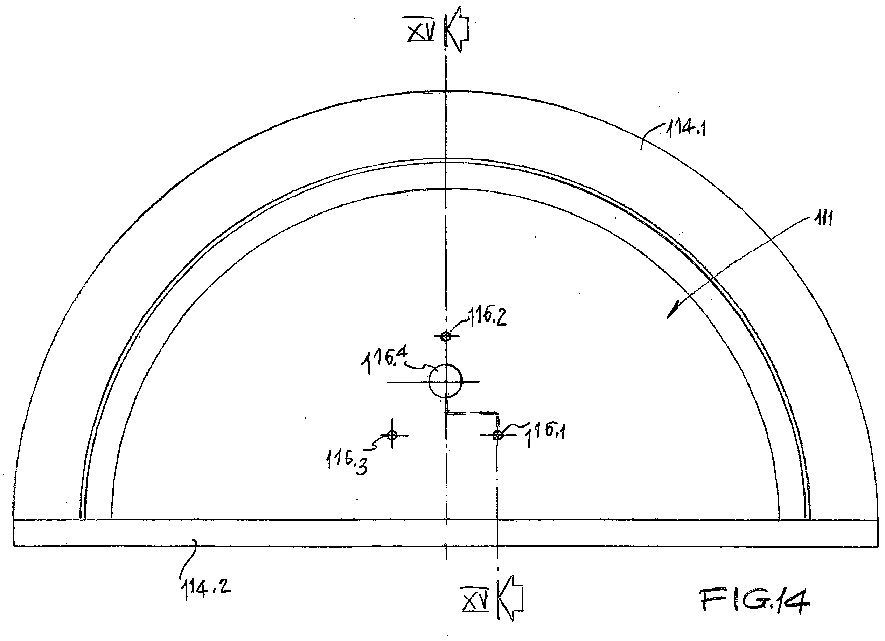

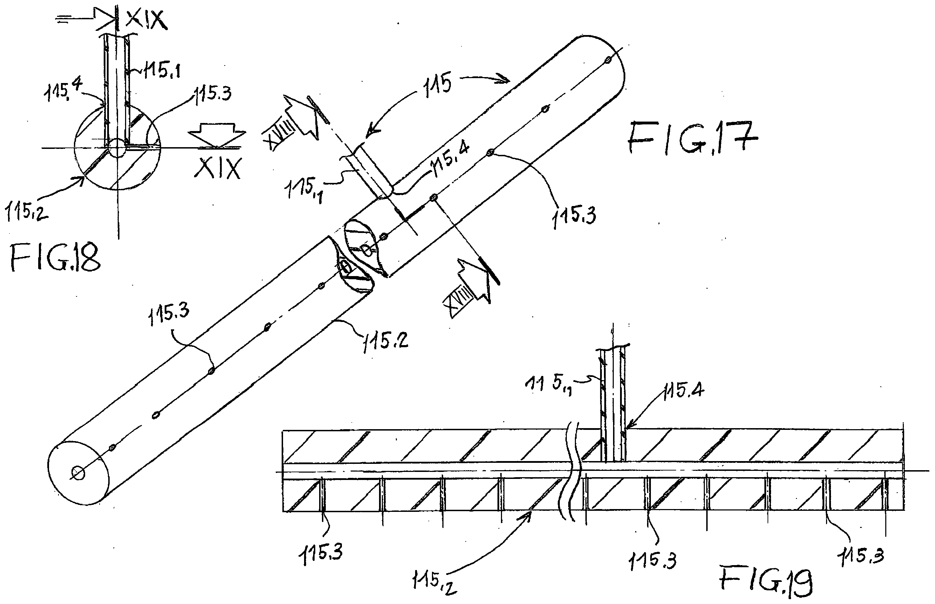

### Second example of an embodiment of the invention (figures 11 to 20)

With reference to the accompanying drawings, the number 110 e is used to refer to the entire dynamic flux chamber for monitoring and sampling indoor soil gas, according to a second example of an embodiment of the present invention. In particular, said flux chamber 110 comprises:
- a body 111 including an internal cavity 112 for collecting soil gas and having a mouth 113 with two contiguous out-turned lips 113.1, 113.2 arranged on two planes substantially orthogonal to one another,
- a seal 114 juxtaposed against said two lips 113.1, 113.2 of said mouth 113;
- a pipe 115 for feeding pressurized inert gas at least into said internal cavity 112, coming from an external source of pressurized inert gas (not illustrated);
- a plurality of apertures in said body 111; specifically: an aperture 116.1 for the inflow of inert gas through said pipe 115, an aperture 116.2 for the outflow of excess inert gas, an aperture 116.3 for parameter control during monitoring and an aperture 116.4 for sampling the test gas collected in said cavity.

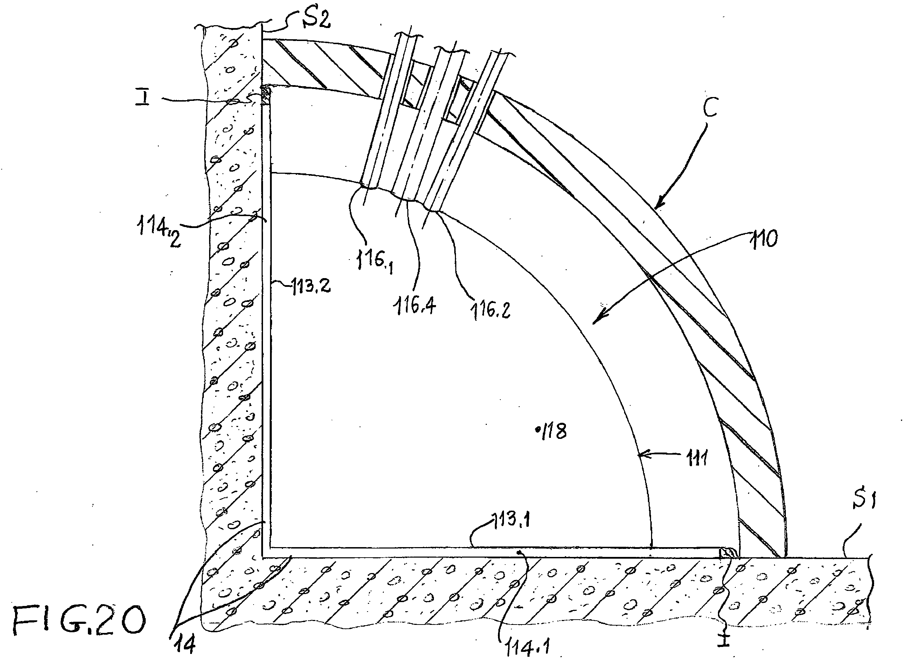

Furthermore, said flux chamber 110 is closed by means of two surfaces S1, S2 external to said chamber 110, contiguous to one another, corresponding to an interstitial space, and substantially orthogonal, against which said seal 114 is juxtaposed (fig. 20).

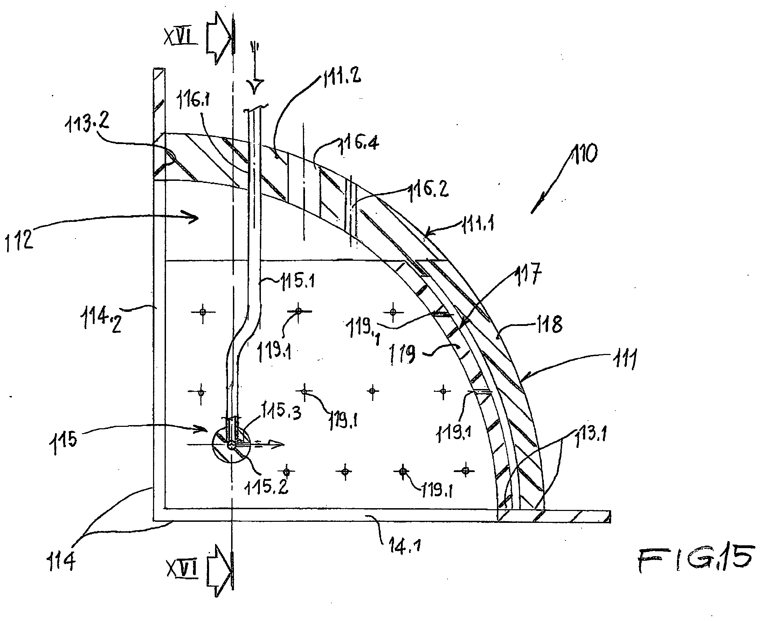

In particular, said dynamic flux chamber 110 comprises a partial gap 117 (fig. 15) provided between an outer wall 118 and an inner wall 119 of said body 111. Said pipe 115 comprises a first duct 115.1 for feeding pressurized inert gas in a gas-tight manner from said external source of pressurized inert gas into said cavity 112 of said body 111 and a second duct 115.2 feeding pressurized inert gas into said cavity 112 and into said gap 117. Said inner wall 119 of said body 111 comprises a plurality of through-holes 119.1, which place in mutual gas communication said gap 117 and said cavity 112.

In particular, said second duct 115.2 connects in a gas-tight manner said first duct 115.1 with said gap 117 and has a plurality of through-holes 115.3 for the passage of pressurized inert gas into said cavity 112 of said body 111.

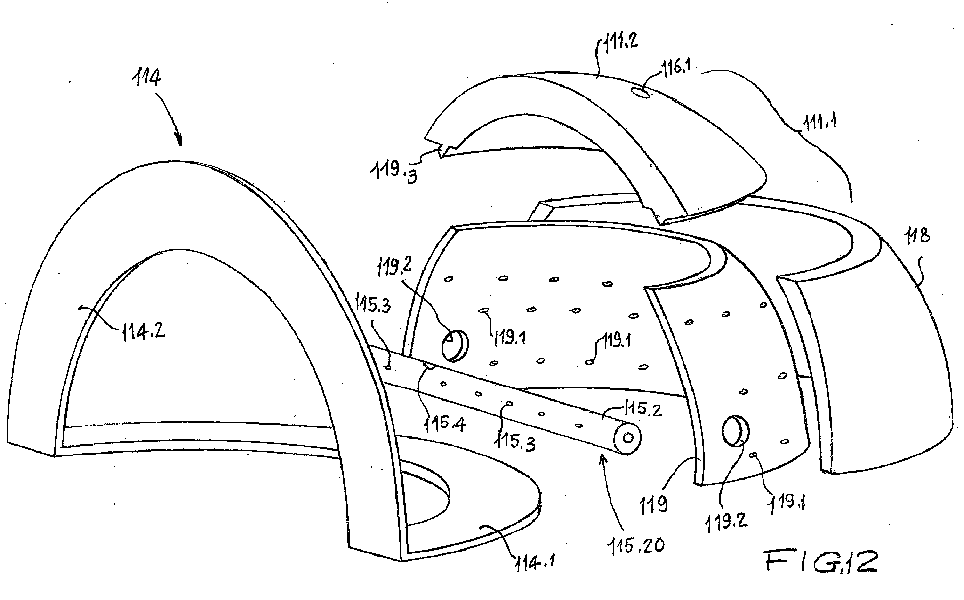

Additionally, said body 111 of said flux chamber 110 comprises (fig. 12):
- a semi-dome 111.1 including:
   - a top cover 111.2, which has said plurality of apertures 116.1, 116.2, 116.3, 116.4;
   - said outer wall 118;
   - said inner wall 119, wherein are provided a plurality of small-diameter through-holes 119.1 and two through-holes 119.2 of a larger diameter and linearly opposite one another;
   - a continuous peripheral projection 119.3 of said cover 111.2, interposed between the upper zones of said outer wall 118 and inner wall 119, which forms a spacer between said walls by having a width substantially corresponding to that of said gap 117;
   - a tubular organ 115.20 comprising said second duct 115.2, having a plurality of small-diameter radial through-holes 115.3 and a gas-tight radial hole 115.4 connecting said first duct 115.1; said tubular organ 115.20 is arranged with its axial ends in said through-holes 119.2 of a larger diameter provided in said inner wall 119 and forming a tunnel for inert gas to flow into said gap 117.

Furthermore, said seal 114 has a frame shape including two wings 114.1, 114.2 substantially orthogonal to one another, of which one 114.1 horizontal and the other 114.2 vertical, and against which rest, respectively, said two out-turned lips 113.1, 113.2 of the mouth 113 of said body 111.

In particular, said two wings 114.1, 114.2 of said frame 114 are curved and said inner wall 119 and said outer wall 118 rest on the horizontal wing 114.1 of said frame 114 and said cover 111.2 rests against the vertical wing 114.2 of said frame 114, while said wings 114.1, 114.2 project in part externally beyond said outer wall 118 and said cover 111.2.

Said body 111 and said frame 114 provide a kit of components, facilitating the commissioning and decommissioning of said flux chamber 110, even by an inexpert person.

### Operation

The flux chamber 110 is operated as illustrated in fig. 20. The seal frame 114 is placed against the two surfaces S1, S2 (the floor and a wall of a closed environment, respectively, at a gap or fissure between them). The body 111 is then placed in contact with said frame 114, juxtaposing the lips 113.1, 113.2 of the mouth 113 against the wings 114.1, 114.2 of said frame.

The flux chamber 110 is partially sealed by placing along said frame 114 inert granular material I not containing any chemical substance potentially present in the test gas. This arrangement creates a barrier to the inert gas that will be insufflated into the chamber 110. Therefore, the inert material I used must not be completely insulating and sealing, for example damp sand could be used.

In this manner, using the flux chamber 110 it is possible to monitor in the gap between the two surfaces S1, S2 the natural leakage points of the test area and therefore the gases present in the subsoil or impregnated in the wall and/or in the floor having the respective surfaces S1 and S2.

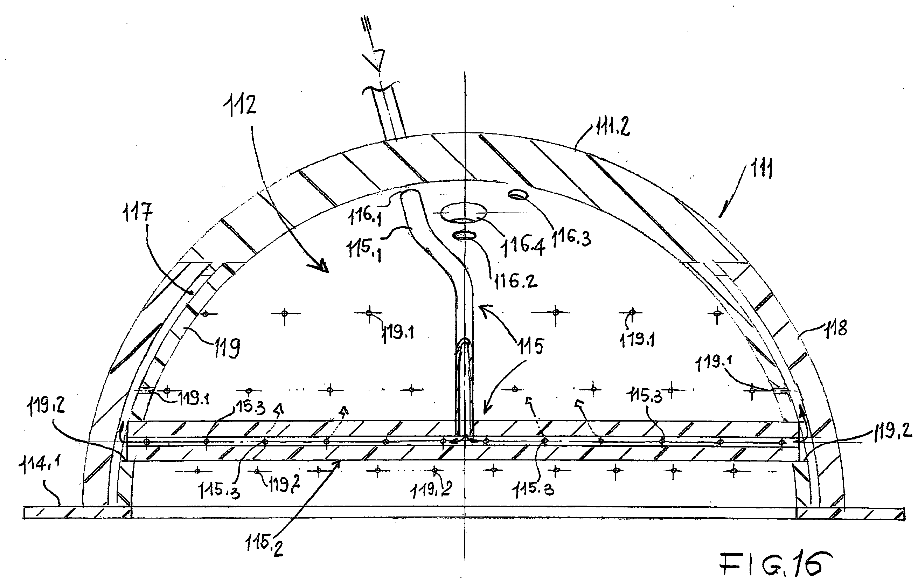

An inert gas, such as gaseous nitrogen, is blown continuously and constantly through the pipe 115, 115.1 (fig. 16) passing through the hole 116.1 in the cover 111.2, into the cavity 112 of the body 111 of the flux chamber 110. in particular, the duct 115.1 of the pipe 115 is connected at its outer end to a pressure reducing valve of a cylinder of pressurized gas, and, at its inner end, to the duct 115.2, constituting a flux tunnel. In this way, the inert gas passes through the flux tunnel 115.2 and begins to flow out from the small holes 115.3 penetrating into the cavity 112 of the flux chamber 110. Residual inert gas then continues towards and as far as the gap 117, which is narrow to guarantee a homogeneous outflow of the inert gas through the small holes 119.1 of the inner wall 119. The cover 111.2, acting as a seal between the two walls 118, 119 of the body 111 of the flux chamber, allows the inert gas to flow out only through said small holes 119.1, penetrating into the internal cavity 112 of the chamber.

The soil gas present in the test area is then extracted and collected inside the cavity 112 of the body 111 of the flux chamber and then sampled in specific substrates, using conventional sampling methods. To this end, testing must be conducted over a sufficiently long period of time, so that the soil gas collects in said cavity 112, joining with the continuously and constantly insufflated inert gas. The test may, for example, last for at least 240 minutes.

In this manner, in the internal cavity 112 of the flux chamber, after a brief period to bleed the flow of inert gas, which is substantially equivalent to four times the internal volume of the cavity 112 of the flux chamber, an atmosphere is obtained with a composition different from the surrounding atmosphere. It is possible to verify whether this condition exists by checking - using an instrument introduced through the aperture 116.3 in the cover 111.2 - the percentage of oxygen present inside the flux chamber 110, which must be near 1% (using an oxygen-free inert gas), for the correct conduct of the analysis.

In this manner, present inside the flux chamber 110 are gaseous nitrogen (an example of an insufflated inert gas) and soil gas, without any influence from the outside environmental gas. It is also possible occasionally to check the pressure, temperature and humidity inside the flux chamber 10 and compare them with those in the surrounding environment, by means of instruments introduced through one or more of the apertures 116.2, 116.3 or 116.4 in the cover 111.2.

To prevent the external temperature, which propagates in a different way to the gas contained in the flux chamber 110, from influencing the internal temperature of the flux chamber, a thermally insulating cover C is placed on it (fig. 20), made for example from a single block of polystyrene, provided with through-holes corresponding to the apertures 116.1, 116.2, 116.3 and 116.4 in the cover 111.2 of the flux chamber 110.

To conduct the test, a special probe draws off part of the gas present in the cavity 112 of the flux chamber 110 through the aperture 116.4 in the cover 111.2, for sampling in suitable substrates, which are subsequently analysed in the laboratory.

The indoor dynamic flux chamber 110, as described above, guarantees the possibility of extracting the gas from a natural gap in a closed environment, and maintenance of an insulated test environment, influenced only by the soil gas, throughout the cavity 112 of the flux chamber.

In this way, testing is not conducted in a permanent or invasive manner in the area of interest; it can be carried out in all conditions and enables a realistic measurement of the soil gas to be obtained.

Procedure for monitoring and sampling soil gas from an indoor or outdoor breathable surface, actuated by means of said dynamic flux chamber 110.

Said procedure comprises the following steps:
- said seal 114 is placed against two essentially orthogonal surfaces S1, S2 of a closed environment, at an interstitial space;
- said body 111 is placed in contact with said seal 114 juxtaposing the lips 113.1, 113.2 of the mouth 113 against the seal;
- an inert gas is blown continuously and constantly through said pipe 115, 115.1, passing through a hole 116.1 of said body 111, 111.2 of the flux chamber 110, into said cavity 112 of the body 111, coming from an external source of pressurized inert gas;
- soil gas present in the test area is extracted, and is collected inside said cavity 112 of the body 111 of the flux chamber and then sampled in specific substrates.

Furthermore, according to the afore-mentioned procedure:
- the inert gas is channelled through said second duct 115.2, provided with small holes 115.3, and partly flows out of said small holes 115.3, penetrating into said cavity 112 of the body 111 of the flux chamber 110,
- residual inert gas is further channelled towards and as far as said gap 117 between said outer wall 118 and said inner wall 119 of said body 111, provided with small holes 119.1, and flows out through said small holes 119.1, penetrating into the internal cavity 112 of the chamber.

As will be apparent from the above description, the present invention provides a dynamic flux chamber for monitoring and sampling soil gas from an indoor or outdoor breathable surface, such as for example green areas, parks, depots or similar.

In particular, said dynamic flux chamber according to the invention can be used for testing sites that are polluted, or thought to be polluted, by characterizing and monitoring the soil gas.

In addition, said dynamic flux chamber according to the invention can be used to measure the concentration of the gases present in the inter-granular spaces of the subsoil, for example according to the provisions of Italian Legislative Decree 152/06, with reference to surfaces even in outdoor environments. Additionally, the present invention provides a procedure for monitoring and sampling soil gas from an indoor or outdoor breathable surface, that is simple, safe and reliable to conduct and can guarantee the insufflation of inert gas in a homogeneous manner throughout the chamber, thereby aiding in the extraction of the soil gas in a homogeneous manner over the entire surface on which the flux chamber is placed, without acting permanently and/or invasively in the testing area, and guaranteeing a realistic measurement of the soil gas.

## Claims

1. Dynamic flux chamber (10, 110) for monitoring and sampling soil gas from an indoor or outdoor breathable surface (S1; S1, S2), comprising:
- a body (11, 111) including an internal cavity (12, 112) for collecting soil gas and having a mouth (13, 113) overlaid on said breathable surface (S1; S1, S2);
- a pipe (15, 115) for feeding pressurized inert gas at least into said internal cavity (12, 112), coming from an external source of pressurized inert gas;
- a plurality of apertures (16.1, 16.2, 16.3, 16.4; 116.1, 116.2, 116.3, 116.4) provided in said body (11, 111), both for the inflow of inert gas through said pipe (15, 115), and for the outflow of excess inert gas and/or for parameter control during monitoring and/or for sampling the test gas collected in said cavity (12, 112), **characterized in that** it comprises a seal (14, 114) juxtaposed against said mouth (13, 113) and resting against said breathable surface (S1; S1, S2).

2. Dynamic flux chamber (10, 110) according to claim 1, **characterized in that** it comprises an at least partial gap (17, 117) provided between an outer wall (18, 118) and an inner wall (19, 119) of said body (11, 111), **in that** said pipe (15, 115) comprises a first duct (15.1, 115.1) for feeding pressurized inert gas branched in an gas-tight manner from said external source of pressurized inert gas into said cavity (12, 112) of said body (11, 111) and a second duct (15.2, 115.2) feeding pressurized inert gas into said cavity (12, 112) and/or into said gap (17, 117), and **in that** said inner wall (19, 119) of said body (11, 111) comprises at least one through-hole (19.1, 119.1), which creates a mutual gas communication between said gap (17, 117) and said cavity (12, 112).

3. Dynamic flux chamber (10, 110) according to claim 2, **characterized in that** said second duct (15.2, 115.2) connects in a gas-tight manner said first duct (15.1, 115.1) with said gap (17, 117) and has at least one through-hole (15.3, 115.3) for the passage of pressurized inert gas into said cavity (12, 112) of said body (11, 111).

4. Dynamic flux chamber (10, 110) according to claim 2 and/or 3, **characterized in that** said body (11, 111) of said flux chamber (10, 110) comprises:
- a dome (11.1, 111.1) including:
- a top cover (11.2, 111.2), which has said plurality of apertures (16.1, 16.2, 16.3, 16.4; 116.1, 116.2, 116.3, 116.4);
- said outer wall (18, 118);
- said inner wall (19, 119), wherein are provided a plurality of small-diameter through-holes (19.1, 119.1) and two through-holes (19.2, 119.2) of a larger diameter;
- a continuous peripheral projection (19.4, 119.3) of said cover (11.2, 111.2), engaged with the upper zone of at least said outer wall (18, 118) and/or said inner wall (19, 119);
- a tubular organ (15.2, 115.20) comprising said second duct (15.2, 115.2), having a plurality of small-diameter radial through-holes (15.3, 115.3) and a gas-tight radial hole (15.4, 115.4) connecting said first duct (15.1, 115.1), said tubular organ (15.2, 115.20) being arranged with its axial ends in said through-holes (19.2, 119.2) of a larger diameter provided in said gas-tight inner wall (19, 119), providing a tunnel for inert gas to flow into said gap (17, 117).

5. Dynamic flux chamber (10) according to one or more of the previous claims, **characterized in that** said seal (14) has a circular frame shape, against which rest at least said inner wall (19, 19.3) and/or said outer wall (18).

6. Dynamic flux chamber (10, 110) according to one or more of the previous claims, **characterized in that** it is lined on the outside with a thermally insulating covering (C) that also blocks UV radiation.

7. Dynamic flux chamber (10, 110) according to one or more of the previous claims, **characterized in that** it comprises inert material (I) not containing any chemical substance present in the test gas, said inert material (I) being arranged along the outer edge of said seal (14, 114), so that said inert material (I) is not completely insulating and sealing.

8. Procedure for monitoring and sampling soil gas from a flat outdoor or indoor breathable surface (S1), actuated by means of the dynamic flux chamber (10) according to one or more of the previous claims, wherein:
- said seal (14) is placed against said flat breathable surface (S1) of an outdoor or indoor environment;
- said body (11) is placed in contact with said seal (14) juxtaposing the edge (19.3) of said mouth (13) against the seal;
- an inert gas is blown continuously through said pipe (15, 15.1, 15.2), passing through a hole (16.1) of said body (11, 11.1, 11.2) of the flux chamber (10), into said cavity (12) of the body, coming from an external source of pressurized inert gas;
- soil gas present in the test area is extracted, and is collected inside said cavity (12) of the body (11) of the flux chamber and then sampled in specific substrates.

9. Procedure for monitoring and sampling soil gas from a flat outdoor or indoor breathable surface (S1) according to claim 8, wherein:
- the inert gas is channelled through said second duct (15.2), provided with small holes (15.3), and partly flows out of said small holes (15.3), penetrating into said cavity (12) of the body (11, 11.1, 11.2) of the flux chamber (10),
- residual inert gas is further channelled towards and as far as said gap (17) between said outer wall (18) and said inner wall (19) of said body (11), provided with small holes (19.1), and flows out through said small holes (19.1), penetrating into the internal cavity (12) of the chamber.

10. Dynamic flux chamber (110) for monitoring and sampling indoor soil gas, comprising:
- a body (111) including an internal cavity (112) for collecting soil gas and having a mouth (113),
- a pipe (115) for feeding pressurized inert gas at least into said internal cavity (112), coming from an external source of pressurized inert gas;
- a plurality of apertures (116.1, 116.2, 116.3, 116.4) provided in said body (111), both for the inflow of inert gas through said pipe (115), and for the outflow of excess inert gas and/or for parameter control during monitoring and/or for sampling the test gas collected in said cavity, **characterized in that** it comprises said mouth (113) configured with two contiguous out-turned lips (113.1, 113.2) arranged on two planes substantially orthogonal to one another, and a seal (114) juxtaposed against said two lips (113.1, 113.2) of said mouth (113), and **in that** said flux chamber (110) is closed by means of two surfaces (S1, S2) external to said chamber (110), contiguous to one another, corresponding to an interstitial space, and substantially orthogonal, against which said seal (114) is juxtaposed.

11. Dynamic flux chamber (110) according to claim 10, **characterized in that** said seal (114) has a frame shape including two wings (114.1, 114.2) substantially orthogonal to one another, of which one (114.1) is horizontal and the other (114.2) vertical, and against which rest, respectively, said two out-turned lips (113.1, 113.2) of said mouth (113) of said body (111) .

12. Dynamic flux chamber (110) according to claim 11, **characterized in that** said two wings (114.1, 114.2) of said frame (114) are curved and said inner wall (119) and said outer wall (118) rest on the horizontal wing (114.1) of said frame (114) and said cover (111.2) rests against the vertical wing (114.2) of said frame (114).

13. Procedure for monitoring and sampling indoor soil gas, actuated by means of the dynamic flux chamber (110) according to claim 10, 11 and/or 12, wherein:
- said seal (114) is placed against two essentially orthogonal surfaces (S1, S2) of a closed environment, at an interstitial space;
- said body (111) is placed in contact with said seal (114) juxtaposing the lips (113.1, 113.2) of the mouth (113) against the seal;
- an inert gas is blown continuously and constantly through said pipe (115, 115.1), passing through a hole (116.1) of said body (111, 111.2) of the flux chamber (110), into said cavity (112) of the body (111), coming from an external source of pressurized inert gas;
- soil gas present in the test area is extracted, and is collected inside said cavity (112) of the body (111) of the flux chamber and then sampled in specific substrates.

14. Procedure for monitoring and sampling indoor soil gas according to claim 13, wherein:
- the inert gas is channelled through said second duct (115.2), provided with small holes (115.3), and partly flows out of said small holes (115.3), penetrating into said cavity (112) of the body (111) of the flux chamber (110),
- residual inert gas is further channelled towards and as far as said gap (117) between said outer wall (18) and said inner wall (119) of said body (111), provided with small holes (119.1), and flows out through said small holes (119.1), penetrating into the internal cavity (112) of the chamber.

15. Kit of components for assembly of the monitoring and sampling chamber according to claim 1 or claim 10.
